# EUROPEAN PATENT APPLICATION

(11) **EP 4 063 351 A1**
(43) Date of publication of application: **28.09.2022**
(21) Application number: 21305384.6
(22) Date of filing: 26.03.2021
(51) Int. Cl.: C07D 215/38

(54) **PREPARATION METHOD OF QUINOLINE DERIVATIVE COMPOUNDS**

(71) Applicant: ABIVAX, 75008 Paris (FR); Centre national de la recherche scientifique, 75016 Paris (FR); UNIVERSITE DE MONTPELLIER, 34090 Montpellier (FR); Institut Curie, 75248 Paris Cedex 05 (FR)
(72) Inventor: DENIS, Jérôme, 75008 Paris (FR); DE BLASIO, Fabien, 78520 Limay (FR); BOYER, Thierry, 92290 Chatenay-Malabry (FR); GUERIN, Charles, 78711 Mantes La Ville (FR); MICHAUX, Julien, 63200 Riom (FR); NAJMAN, Romain, 94240 L'Hay Les Roses (FR); MAHUTEAU-BETZER, Florence, 78470 Saint Remy-Les-Chevreuse (FR)
(74) Representative: Nony

(57) **Abstract**

The present invention relates to a method for preparing a compound of formula (I) comprising the following steps: (i) reacting a compound of formula (II) with a compound of formula (III), to form the hydrochloride salt of the compound of formula (I), wherein the molar ratio of the compound of formula (II) vs. the compound of formula (III) is from 1.00:0.80 to 1.00:1.20, and no metal catalyst is present, and (ii) recovering the compound of formula (I) in the form of a free base through addition of a base.

## Description

### FIELD OF THE INVENTION

The present invention relates to a method for preparing quinoline derivatives.

### BACKGROUND OF THE INVENTION

WO2010/143169 application describes the preparation and use of compounds, and in particular quinoline derivatives useful in the treatment of HIV infection. Said application in particular discloses 8-chloro-N-(4-(trifluoromethoxy)phenyl)quinolin-2-amine also named (8-chloro-quinoline-2-yl)-(4-trifluoromethoxy-phenyl)-amine. Said compound is also known as ABX464, which is currently under clinical development.

A route of synthesis is disclosed in said patent application implementing a coupling step using a Buchwald-Hartwig amination in the presence of palladium acetate and Xantphos. Example 5 of WO2010/143169 application is namely illustrating this route of synthesis to yield 8-chloro-N-(4-(trifluoromethoxy)phenyl)quinolin-2-amine, corresponding to compound (90) in said document.

WO2017/158201 application deals with a process for preparing quinolin-2-yl-phenylamine derivatives and their salts by implementing a coupling step using an aniline derivative in excess and no metal catalyst. In the preferred embodiments of the application, 2-3 moles of aniline derivative is used per mole of quinoline derivative. As described on page 5 and illustrated in Example 3 of WO2017/158201**,** the second equivalent of aniline derivative is required to serve as a base in order to allow direct isolation of the free base of 8-chloro-N-(4-(trifluoromethoxy)phenyl)quinolin-2-amine. However, the use of an aniline derivative in large excess is expensive and also detrimental towards the environment as it is used to neutralize the released hydrochloric acid and not completely consumed during the process in case of more than 2 equivalents engaged in the step of process for preparing quinoline-2-yl-phenylamine derivatives. Other advantages with respect to this prior art, in connection to the industrial scale constraint, are detailed herein after.

Therefore, there is still a need to provide a manufacturing process compliant with an industrial scale production.

### SUMMARY OF THE INVENTION

The present invention is intended to provide a method for preparing quinoline derivative compounds which is economical due to the use of inexpensive reagents, is more environment-friendly and exhibits an excellent product yield and is therefore suitable for industrial-scale mass production.

For the preparation of 8-chloro-N-(4-(trifluoromethoxy)phenyl)quinolin-2-amine, in particular, the requirements of WO2017/158201 for 2-3 equivalents of aniline derivative is avoided in the present invention by first preparing the hydrochloride salt of 8-chloro-N-(4-(trifluoromethoxy)phenyl)quinolin-2-amine, then preparing the free base using an inexpensive base. In preferred embodiments of the present invention, the hydrochloride salt of 8-chloro-N-(4-(trifluoromethoxy)phenyl)quinolin-2-amine is isolated prior to preparing the free base. This isolation affords the hydrochloride salt of compound of formula (I) in high purity prior to preparing the free base, compared to manufacturing processes implementing aniline derivatives in excess, which gives crude compound of formula (I) while requiring a step of purification by recrystallization. This manufacturing process of said prior art has moreover the disadvantage of necessitating large volumes of solvents because of the formation of hard block during the coupling step, comprising, *inter alia,* the by-product aniline hydrochloride. Namely, elimination of said by-product requires purification treatment involving large volumes of solvents due to hard solubilization. The aniline in excess also requires a purification treatment involving large volumes of solvents. For obvious reasons, to an industrial scale, it is not desirable to use such large volumes of solvents at any one time in the manufacturing process.

The need for a palladium-scavenging step is among all avoided as far as the present preparation method is carried out in the absence of a Palladium catalyst. In addition, the purity of the obtained compounds may be controlled better in comparison to known processes by the implementation of controlled steps prior to the final coupling step as it will be detailed hereinafter.

The present invention provides a method for preparing a compound of formula (I) 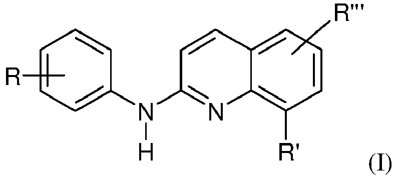 wherein
R is selected from a (C₁-C₃)alkyl group, in particular a methyl group, a (C₁-C₃)alkoxy group, in particular a methoxy group, a (C₁-C₃)fluoroalkyl group, in particular a trifluoromethyl group, a halogen atom and more particularly a fluorine or chlorine atom, a (C₁-C₃)fluoroalkoxy group, in particular a trifluoromethoxy group and a -NR₁R₂ group, in particular an amino group,
R' represents a halogen atom and more particularly a fluorine or chlorine atom or a methyl group, and
R"' represents a hydrogen atom or a 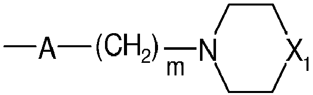 group, wherein A is O or NH, m is 2 or 3 and X₁ is -O-, -CH₂- or -N(Ra)-, where Ra is a (C₁-C₃)alkyl group, in particular a methyl group, and
R₁ and R₂ are independently a hydrogen atom or a (C₁-C₃)alkyl group, the method comprising the following steps:
   - (i) reacting a compound of formula (II) 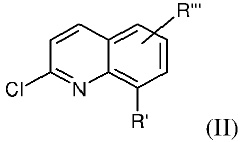 wherein R' and R'" are as defined above,
      with a compound of formula (III) 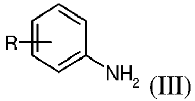 wherein R is as defined above,
      to form the hydrochloride salt of the compound of formula (I),
      wherein the molar ratio of the compound of formula (II) vs. the compound of formula (III) is from 1.00 :0.80 to 1.00:1.20, and no metal catalyst is present and then
   - (ii) recovering the compound of formula (I) in the form of a free base through addition of a base.

One of ordinary skill in the art will appreciate that the compound of formula (I), as prepared by the methods of the present invention, may be further treated with a suitable acid to form a salt thereof, according to conventional methods of organic synthesis.

The present invention further relates to a method of manufacturing of a compound of formula (I) as described above further comprising the step of preparing a pharmaceutical composition comprising such compound of formula (I), with pharmaceutically acceptable excipients.

As used herein, the term "ambient temperature" or "room temperature" refers to a temperature ranging from 15°C to 30°C, more particularly from 18°C to 25°C.

### BRIEF DESCRIPTION OF THE FIGURES

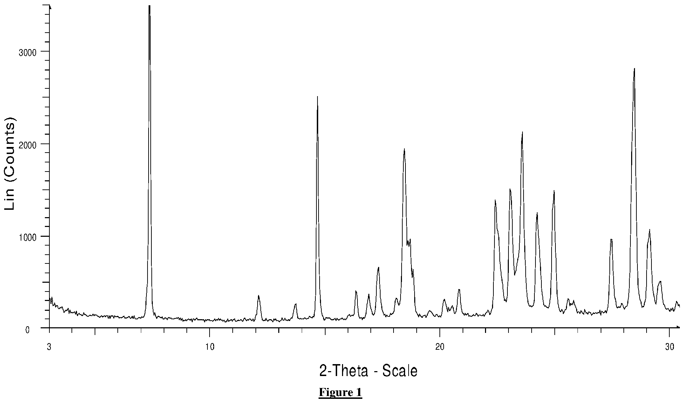

Figure 1 is a X-ray powder diagram of the stable polymorphic form (Form I) of 8-chloro-N-(4-(trifluoromethoxy)phenyl)quinolin-2-amine or ABX464.

### DETAILED DESCRIPTION OF THE INVENTION

### Method of preparation of compound of formula (I)

The present invention provides a method for preparing a compound of formula (I) 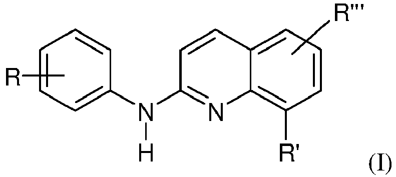 wherein
R is selected from a (C₁-C₃)alkyl group, in particular a methyl group, a (C₁-C₃)alkoxy group, in particular a methoxy group, a (C₁-C₃)fluoroalkyl group, in particular a trifluoromethyl group, a halogen atom and more particularly a fluorine or chlorine atom, a (C₁-C₃)fluoroalkoxy group, in particular a trifluoromethoxy group and a -NR₁R₂ group, in particular an amino group,
R' represents a halogen atom and more particularly a fluorine or chlorine atom or a methyl group, and
R"' represents a hydrogen atom or a 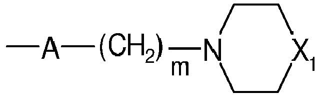 group, wherein A is O or NH, m is 2 or 3 and X₁ is -O-, -CH₂- or -N(Ra)-, where Ra is a (C₁-C₃)alkyl group, in particular a methyl group, and
R₁ and R₂ are independently a hydrogen atom or a (C₁-C₃)alkyl group, the method comprising the following steps:
   - (i) reacting a compound of formula (II) 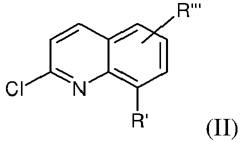 wherein R' and R'" are as defined above,
      with a compound of formula (III) 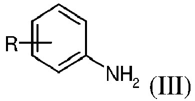 wherein R is as defined above,
      to form the hydrochloride salt of the compound of formula (I),
      wherein the molar ratio of the compound of formula (II) vs. the compound of formula (III) is from 1.00 :0.80 to 1.00:1.20, and no metal catalyst is present and then
   - (ii) recovering the compound of formula (I) in the form of a free base through addition of a base.

As used herein, the reaction of the compound of formula (II) with the compound of formula (III), i.e. step (i) is also called a coupling step. More particularly, the coupling step (i) is a nucleophilic aromatic substitution.

In preferred embodiments, the method further comprises the step of isolating the hydrochloride salt of the compound of formula (I), between step (i) and (ii). This allows a better purification of the final compound of formula (I). In other words, potential impurities may be removed more easily after the coupling step (i), in particular in comparison to manufacturing processes implementing aniline derivatives in excess.

For this reason, as well as for the reasons already mentioned above, the present method for preparing a compound of formula (I) is thus particularly suitable for an industrial scale manufacture.

According to a preferred embodiment, the present invention relates to a method for preparing a compound of formula (I) 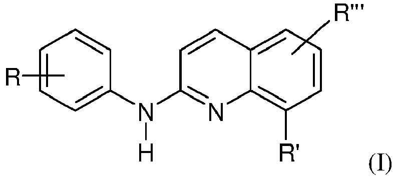 wherein
R is a (C₁-C₃)fluoroalkoxy group, in particular a trifluoromethoxy group,
R' represents a halogen atom and more particularly a fluorine or chlorine atom, and
R"' represents a hydrogen atom,
the method comprising the following steps:
   - (i) reacting a compound of formula (II) 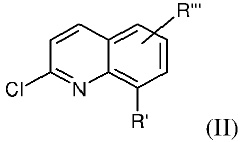 wherein R' represents a halogen atom and more particularly a fluorine or chlorine atom, and R"' represents a hydrogen atom,
      with a compound of formula (III) 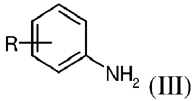 wherein R is a (C₁-C₃)fluoroalkoxy group, in particular a trifluoromethoxy group,
      to form the hydrochloride salt of the compound of formula (I),
      wherein the molar ratio of the compound of formula (II) vs. the compound of formula (III) is from 1.00 :0.80 to 1.00:1.20, and no metal catalyst is present and then
   - (ii) recovering the compound of formula (I) in the form of a free base through addition of a base;
      wherein the hydrochloride salt of the compound of formula (I) is isolated between step (i) and (ii).

According to a particular embodiment, R is in the para position of the phenyl ring with respect to the NH₂ group in a compound of formula (III) as defined in the present invention.

In the context of the present invention, the term:
- "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio,
- "step (i)" and "step (ii)" also refer respectively to the step (i) and the step (ii) comprised in step (E) as defined in the present invention herein after,
- "halogen" is understood to mean chlorine, fluorine, bromine, or iodine, and in particular denotes chlorine, fluorine or bromine,
- "(C₁-C₃)alkyl" as used herein respectively refers to a linear or branched C₁-C₃ alkyl. Examples are methyl, ethyl, n-propyl, and isopropyl,
- "(C₁-C₃)alkoxy" as used herein respectively refers to O-(C₁-C₃)alkyl moiety, wherein alkyl is as defined above. Examples are methoxy, ethoxy, n-propoxy, and isopropoxy, and
- "fluoroalkyl group" and "fluoroalkoxy group" refers respectively to alkyl group and alkoxy group as above-defined, said groups being substituted by at least one fluorine atom. Examples are perfluoroalkyl groups, such as a trifluoromethyl group and the like or perfluoropropyl group or perfluoroalkoxy group, such as a trifluoromethoxy group and the like.

The compounds of formula (I) can comprise one or more asymmetric carbon atoms. They can thus exist in the form of enantiomers or of diastereoisomers. These enantiomers, diastereoisomers and their mixtures, including the racemic mixtures, may be prepared according to the manufacturing process according to the present invention.

The compounds of formula (I) can be obtained under a free base form, or under a pharmaceutically acceptable polymorphic form (that is to say a crystalline form).

As mentioned above, the molar ratio of the compound of formula (II) vs. the compound of formula (III) may be from 1.00:0.80 (= 1.25) to 1.00:1.20 (= 0.83) and can be for example 1.00:0.80, 1.00:0.85 (= 1.18), 1.00:0.90 (= 1.11), 1.00: 0.95 (=1.05), 1.00:1.00 (= 1), 1.00:1.05 (= 0.95), 1.00:1.10 (= 0.91), 1.00:1.15 (= 0.87) or 1.00: 1.20 (= 0.83).

According to a particular embodiment, the molar ratio of the compound of formula (II) vs. the compound of formula (III) may be from 1.00:0.80 to 1.00:1.10. Thus, preferably, the molar ratio of the compound of formula (II) vs. the compound of formula (III) is for example 1.00:0.80, 1.00:0.85, 1.00:0.90, 1.00: 0.95, 1.00:1.00, 1.00:1.05, or 1.00:1.10.

In a particular embodiment, the method of preparation according to the present invention is carried out in equimolarity conditions with respect to the compounds of formula (II) and (III).

As mentioned above, the process according to the present invention is also characterized in that no metal catalyst is used in the coupling step (i).

Coupling step (i) may be carried out in a solvent. Said solvent used in the coupling step may be an organic solvent which is classically used for performing nucleophilic substitution. For example, said organic solvent may be selected from the group consisting of ethyl acetate, isopropyl acetate, toluene, N,N-dimethylformamide (DMF), N-methyl-2-pyrrolidone (NMP), a (C₁-C₄)alcohol such as methanol, ethanol, isopropanol or isopropyl alcohol (IUPAC name: propan-2-ol, also named IPA) and butanol and the like, and mixtures of the foregoing, and is more particularly selected from the group consisting of ethanol, butanol and isopropanol, and is even more preferably isopropanol.

The coupling step may be carried out over a wide temperature range which does not cause side reactions, but is carried out at a reaction temperature ranging from 60°C to 120°C, preferably from 70°C to 100°C, and most preferably from 80°C to 85°C.

The coupling step may be carried out during from 8 hours to 30 hours, in particular from 10 to 20 hours, and even more particularly from 10 to 16 hours, for example from 10 to 14 hours. Various heating and cooling ramps may be applied, which may be applied according to the common knowledge at the beginning or end, respectively, of the coupling step (i). Each heating and cooling ramp may last between 1 hour and 6 hours.

According to one aspect, the coupling step may be carried out under an inert atmosphere, for example under nitrogen atmosphere.

Herein is further provided a particular embodiment, wherein hydrochloric acid (HCl) is added during step (i).

The inventors have indeed surprisingly found that adding hydrochloric acid at this stage of the method for preparing a compound of formula (I) as defined above affords increased yield.

Hydrochloric acid may be added anytime during step (i), for example during the second half of the duration of step (i), in particular during the last quarter of the duration of step (i) and more particularly during the last eighth of the duration of step (i), for example at the end of step (i) prior to step (ii), as defined above. Hydrochloric acid may typically be added after the cooling ramp has been carried out.

Still according to this particular embodiment, the molar ratio of hydrochloric acid vs. the compound of formula (II) at the beginning of the step as defined above may be from 0.05:1.00 to 0.60:1.00, in particular from 0.05:1.00 to 0.50:1.00, and more particularly from 0.10:1.00 to 0.30:1.00 and can for example be 0.05:1.00, 0.10:1.00, 0.20:1.00, 0.30:1.00, 0.40:1.00 or 0.50:1.00.

Still in connection to particular embodiments when carrying out step (i), enhancement of crystallisation may be advantageous performed.

To this end, it is further provided a particular embodiment, wherein seeds of the hydrochloride salt of the compound of formula (I) are added during step (i).

Seeds of the hydrochloride salt of the compound of formula (I) may be added during step (i), for example as soon as at least 60%, in particular at least 70%, more particularly at least 80% of the maximum theoretical yield of hydrochloride salt of compound of formula (I) is formed. Crystallization of the reaction product, i.e. hydrochloride salt of the compound of formula (I), may be observed as soon as the seeds are added, for example in the form of needles.

Still according to this particular embodiment, the amount of said seeds of hydrochloride salt of the compound of formula (I) may be from 0.05% to 1%, in particular from 0.05% to 0.2%, and even more particularly from 0.1% to 0.2% by weight, with respect to the weight of compound of formula (II) at the beginning of the step as defined above.

Herein is further provided a particular embodiment, wherein an acid is added near the beginning of step (i) as defined above.

The inventors have indeed surprisingly found that the presence of an acid at the beginning of the coupling reaction favourably impacts the kinetic of said step (i). Addition of an acid near the beginning of the reaction leads to a faster reaction rate in comparison to a reaction performed without added acid near the beginning of the reaction, but both conditions afford a similar final reaction conversion. This beneficial effect of adding acid near the beginning of step (i) is particularly surprising in view of previous reports, for example, in WO2017/158201**,** of the importance of including in a coupling reaction an excess of an aniline, which serves as a base.

Said acid may be selected from hydrochloric acid (HCl), hydrobromic acid (HBr), sulfuric acid (H₂SO₄), perchloric acid (HClO₄), phosphoric acid (H₃PO₄), trifluoroacetic acid (TFA), acetic acid, citric acid, oxalic acid, maleic acid, tartaric acid, succinic acid, malonic acid and mixtures thereof, in particular from phosphoric acid (H3PO4), hydrochloric acid (HCl), trifluoroacetic acid (TFA) and mixtures thereof. When hydrochloric acid is implemented, it may be under the form of gaseous hydrochloric acid dissolved in isopropanol.

Still according to this particular embodiment, the molar ratio of acid vs. the compound of formula (II) at the beginning of the step may be from 0.01:1.00 to 0.60:1.00, in particular from 0.05:1.00 to 0.50:1.00, more particularly from 0.10:1.00 to 0.50:1.00 and can for example be 0.10:1.00, 0.20:1.00, 0.30:1.00, 0.40:1.00 or 0.50:1.00.

The acid added near the beginning of step (i) may be added, for example during the first quarter of the duration of step (i) and more particularly during the first eighth of the duration of step (i), for example at the beginning of step (i), as defined above.

Said three particular embodiments as described above in connection to the implementation of step (i) may be performed separately or in combination.

The base used for recovering compound of formula (I) in step (ii) may be a base commonly used at industrial scale. In one embodiment, said base is inexpensive and classically used in manufacturing processes compliant with an industrial scale production. Namely, said base may be selected from a group consisting of an organic base such as pyridine, triethylamine, diisopropylamine and the like, and an inorganic base such as sodium carbonate (Na₂CO₃), sodium bicarbonate (NaHCO₃), potassium carbonate (K₂CO₃), potassium bicarbonate (KHCO₃), sodium hydroxide (NaOH), potassium hydroxide (KOH), lithium hydroxide (LiOH), and the like, in particular an inorganic base such as sodium carbonate (Na₂CO₃), sodium bicarbonate (NaHCO₃), potassium carbonate (K2CO3), sodium hydroxide (NaOH), potassium hydroxide (KOH), and the like. In step (ii), the base is not a compound of formula (II) or formula (III). The foregoing description of the base in step (ii) also applies to part (ii) of step (E), and to step (2), of the methods described herein.

According to a particular embodiment of the present invention, said base is an inorganic base selected from potassium carbonate and sodium carbonate, preferably sodium carbonate.

The molar ratio of the base vs. the compound of formula (II) may in particular range between 0.5 and 3.0, in particular between 1.0 and 2.0, and more particularly between 1.1 and 1.5.

A further purification step may be carried out following step (ii) according to usual methods well known to the man skilled in the art. For example, a filtration may be carried out, for example on celite, for example using the same suspension solvent as described above, more particularly ethyl acetate. Solvent-solvent extractions may also be used as well.

It follows that according to a particular aspect, the method of preparation according to the present invention further comprises purification steps, and for example filtration steps between step (i) and step (ii). In some embodiments, the method further comprises the step of isolating the hydrochloride salt of the compound of formula (I), between step (i) and (ii). In preferred embodiments, the method further comprises the step of isolating the hydrochloride salt of the compound of formula (I) by filtration, between steps (i) and (ii).

According to a specific aspect, the present invention relates to a method of preparation of a compound of formula (I) as defined above, wherein the coupling step is carried out in a molar ratio of the compound of formula (II) vs. the compound of formula (III) ranging from 1.00:0.80 to 1.00:1.10, in a range of temperature between 70°C and 100°C, for 10 to 20 hours, for example 10 to 16 hours, and in an organic solvent selected from ethyl acetate, isopropyl acetate, toluene, N,N-dimethylformamide (DMF), N-methyl-2-pyrrolidone (NMP), (C₁-C₄)alcohol such as methanol, ethanol, isopropanol and butanol and the like, and mixtures of the foregoing, more particularly selected from the group consisting of ethanol, butanol and isopropanol, and which is even more preferably isopropanol.

According to a first variant, the present invention relates to a method of preparation of a compound of formula (I) as defined above, wherein the coupling step is carried out in a 1.00:0.80 molar ratio of the compound of formula (II) vs. the compound of formula (III), in a range of temperature between 70°C and 100°C, for 10 to 20 hours and in an organic solvent selected from ethyl acetate, isopropyl acetate, toluene, N,N-dimethylformamide (DMF), N-methyl-2-pyrrolidone (NMP), (C₁-C₄)alcohol such as methanol, ethanol, isopropanol and butanol and the like, more particularly selected from the group consisting of ethanol, butanol and isopropanol, and which is even more preferably isopropanol.

According to a particular embodiment of this first variant, the present invention relates to a method of preparation of a compound of formula (I) as defined above, wherein the coupling step is carried out in a 1.00:0.80 molar ratio of the compound of formula (II) vs. the compound of formula (III), in a range of temperature between 80°C and 85°C, for 10 to 16 hours and in an organic solvent which is a (C₁-C₄)alcohol such as methanol, ethanol, isopropanol or butanol, and the like and more particularly in ethanol, isopropanol or butanol, and more preferably in isopropanol.

According to a second variant, the present invention relates to a method of preparation of a compound of formula (I) as defined above, wherein the coupling step is carried out in a 1.00:0.90 molar ratio of the compound of formula (II) vs. the compound of formula (III), in a range of temperature between 70°C and 100°C , for 10 to 20 hours and in an organic solvent selected from ethyl acetate, isopropyl acetate, toluene, N,N-dimethylformamide (DMF), N-methyl-2-pyrrolidone (NMP), (C₁-C₄)alcohol such as methanol, ethanol, isopropanol and butanol and the like, more particularly selected from the group consisting of ethanol, butanol and isopropanol, and which is even more preferably isopropanol.

According to a particular embodiment of this second variant, the present invention relates to a method of preparation of a compound of formula (I) as defined above, wherein the coupling step is carried out in a 1.00:0.90 molar ratio of the compound of formula (II) vs. the compound of formula (III), in a range of temperature between 80°C and 85°C, for 10 to 16 hours and in an organic solvent which is a (C₁-C₄)alcohol such as methanol, ethanol, isopropanol or butanol, and the like and more particularly in ethanol, isopropanol or butanol, and more preferably in isopropanol.

According to a third variant, the present invention relates to a method of preparation of a compound of formula (I) as defined above, wherein the coupling step is carried out in a 1.00:0.95 molar ratio of the compound of formula (II) vs. the compound of formula (III), in a range of temperature between 70°C and 100°C , for 10 to 20 hours and in an organic solvent selected from ethyl acetate, isopropyl acetate, toluene, N,N-dimethylformamide (DMF), N-methyl-2-pyrrolidone (NMP), (C₁-C₄)alcohol such as methanol, ethanol, isopropanol and butanol and the like, more particularly selected from the group consisting of ethanol, butanol and isopropanol, and which is even more preferably isopropanol.

According to a particular embodiment of this third variant, the present invention relates to a method of preparation of a compound of formula (I) as defined above, wherein the coupling step is carried out in a 1.00:0.95 molar ratio of the compound of formula (II) vs. the compound of formula (III), in a range of temperature between 80°C and 85°C, for 10 to 16 hours and in an organic solvent which is a (C₁-C₄)alcohol such as methanol, ethanol, isopropanol or butanol, and the like and more particularly in ethanol, isopropanol or butanol, and more preferably in isopropanol.

According to a fourth variant, the present invention relates to a method of preparation of a compound of formula (I) as defined above, wherein the coupling step is carried out in a 1.00:1.00 molar ratio of the compound of formula (II) vs. the compound of formula (III), in a range of temperature between 70°C and 100°C , for 10 to 20 hours and in an organic solvent selected from ethyl acetate, isopropyl acetate, toluene, N,N-dimethylformamide (DMF), N-methyl-2-pyrrolidone (NMP), (C₁-C₄)alcohol such as methanol, ethanol, isopropanol and butanol and the like, more particularly selected from the group consisting of ethanol, butanol and isopropanol, and which is even more preferably isopropanol.

According to a particular embodiment of this fourth variant, the present invention relates to a method of preparation of a compound of formula (I) as defined above, wherein the coupling step is carried out in a 1.00:1.00 molar ratio of the compound of formula (II) vs. the compound of formula (III), in a range of temperature between 80°C and 85°C, for 10 to 16 hours and in an organic solvent which is a (C₁-C₄)alcohol such as methanol, ethanol, isopropanol or butanol, and the like and more particularly in ethanol, isopropanol or butanol, and more preferably in isopropanol.

According to a fifth variant, the present invention relates to a method of preparation of a compound of formula (I) as defined above, wherein the coupling step is carried out in a 1.00:1.10 molar ratio of the compound of formula (II) vs. the compound of formula (III), in a range of temperature between 70°C and 100°C , for 10 to 20 hours and in an organic solvent selected from ethyl acetate, isopropyl acetate, toluene, N,N-dimethylformamide (DMF), N-methyl-2-pyrrolidone (NMP), (C₁-C₄)alcohol such as methanol, ethanol, isopropanol and butanol and the like, more particularly selected from the group consisting of ethanol, butanol and isopropanol, and which is even more preferably isopropanol.

According to a particular embodiment of this fifth variant, the present invention relates to a method of preparation of a compound of formula (I) as defined above, wherein the coupling step is carried out in a 1.00:1.10 molar ratio of the compound of formula (II) vs. the compound of formula (III), in a range of temperature between 80°C and 85°C , for 10 to 16 hours and in an organic solvent which is a (C₁-C₄)alcohol such as methanol, ethanol, isopropanol or butanol and the like and more particularly in ethanol, isopropanol or butanol, and more preferably in isopropanol.

The addition of hydrochloric acid during step (i), of seeds of the hydrochloride salt of the compound of formula (I) during step (i) and/or of an acid near the beginning of step (i) as described above, and more particularly of the three additions in combination, may be performed in each of the above-described particular embodiments.

In the present invention, a compound of formula (II) may be prepared by chlorination of a compound of formula (IV) 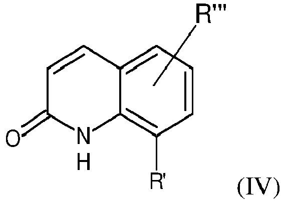 wherein R' and R'" are as defined above.
This step of chlorination (also corresponding to step (D) as defined below) may be carried out by using a chlorination agent selected from a group consisting of thionyl chloride (SOCl₂), PCl₃, POCl₃, PCl₅, and the like, preferably POCl₃, and optionally by using a solvent such as ethyl acetate, acetonitrile, toluene, dichloromethane.

In the present invention, a compound of formula (IV) may be prepared by cyclizing a compound of formula (V) 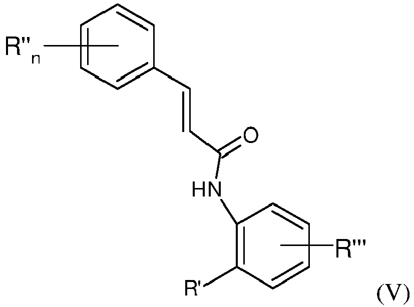 wherein R', R", R'" and n are as defined above.
This step of cyclizing (also corresponding to step (C) as defined below) may be carried out by using chlorobenzene, trifluorotoluene, fluorobenzene, toluene, dichloroethane or the like, and mixtures of the foregoing, and a Lewis acid selected from aluminum chloride (AlCl₃), BF₃, trifluoromethanesulfonic acid, titanium chloride, methanesulfonic acid, and the like, preferably AlCl₃.

In the present invention, a compound of formula (V) may be prepared by amidation of a compound of formula (VI) 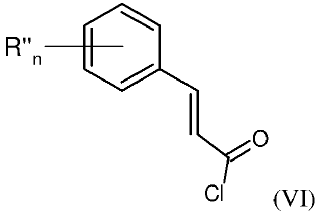 wherein R" and n are as defined above,
with a compound of formula (VI') 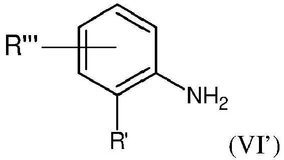 wherein R' and R'" are as defined above.
This step of amidation (also corresponding to step (B) as defined below) may be carried out by using a base selected from a group consisting of an organic base such as pyridine, triethylamine, diisopropylamine and the like, and an inorganic base such as sodium carbonate (Na₂CO₃), sodium bicarbonate (NaHCO₃), potassium carbonate (K2CO3), potassium bicarbonate (KHCO₃), sodium hydroxide (NaOH), potassium hydroxide (KOH), lithium hydroxide (LiOH), and the like, in particular an inorganic base such as sodium carbonate (Na₂CO₃), sodium bicarbonate (NaHCO₃), potassium carbonate (K2CO3), sodium hydroxide (NaOH), potassium hydroxide (KOH), and the like, and preferably potassium carbonate.

In the present invention, a compound of formula (VI) may be prepared by conversion of a carboxylic acid function of a compound of formula (VII) 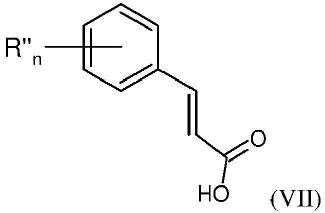 wherein R" and n are as defined above, into an acyl chloride function.
This step of conversion (also corresponding to step (A) as defined below) may be carried out by using a chlorination agent selected from a group consisting of thionyl chloride (SOCl₂), PCl₃, POCl₃, PCl₅, and the like, preferably SOCl₂ and optionally in a solvent such as dichloromethane.

Thus, herein is further provided a method for preparing a compound of formula (I) as defined in the present invention wherein it comprises at least the following steps:
- Step (A): conversion of a carboxylic acid function of a compound of formula (VII) 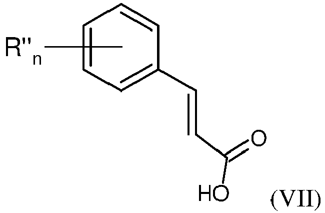 wherein R" and n are as defined above,
   into an acyl chloride function,
   to prepare a compound of formula (VI) 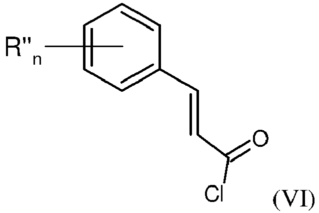 wherein R" and n are as defined above;
- Step (B): amidation of a compound of formula (VI) 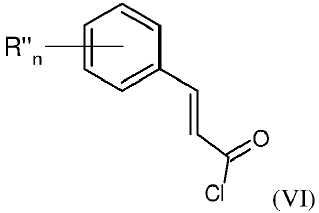 wherein R" and n are as defined above,
   with a compound of formula (VI') 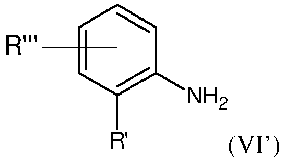 wherein R' and R'" are as defined above,
   to prepare a compound of formula (V) 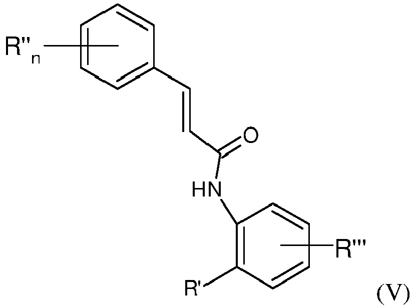 wherein R', R"', R" and n are as defined above;
- Step (C): cyclizing a compound of formula (V) 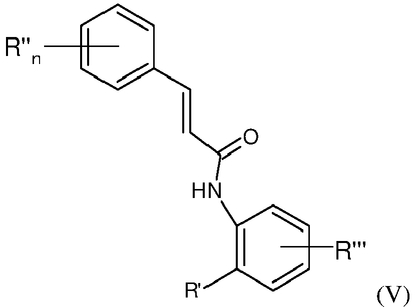 wherein R', R"', R" and n are as defined above,
   to prepare a compound of formula (IV) 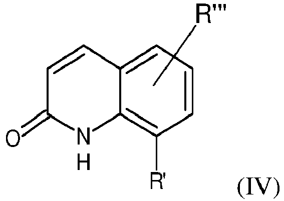 wherein R' and R'" are as defined above,
- Step (D): chlorination of a compound of formula (IV) 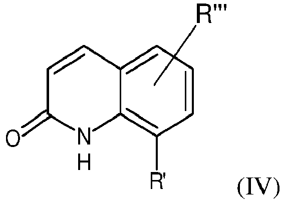 wherein R' and R'" are as defined above,
   to prepare a compound of formula (II) 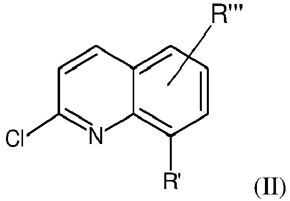 wherein R' and R'" are as defined above,
- Step (E):
   ∘ (i) reacting a compound of formula (II) 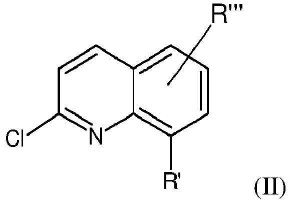 wherein R' and R'" are as defined above,
      with a compound of formula (III) 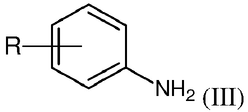 wherein R is as defined above,
      to form the hydrochloride salt of the compound of formula (I),
      wherein the molar ratio of the compound of formula (II) vs. the compound of formula (III) is from 1.00:0.80 to 1.00:1.20, and no metal catalyst is present, and then
   ∘ (ii) recovering the compound of formula (I) in the form of a free base through addition of a base.

The addition of hydrochloric acid during part (i), of seeds of the hydrochloride salt of the compound of formula (I) during part (i) and/or of an acid near the beginning of part (i) as described above, and more particularly of the three additions in combination, may be performed in part (i) of step (E) as described above.

An illustration of the preparation method of the present invention with appropriate conditions is provided in example 1, steps 1 to 4.

An illustration of the coupling step (i) implementing the addition of hydrochloric acid during step (i), of seeds of the hydrochloride salt of the compound of formula (I) during step (i) and/or of an acid near the beginning of step (i) as described above is provided in examples 2 and 3.

In the present invention, compound of formula (III) is commercially available or may be prepared according to methods known by the man skilled in the art.

The preparation method according to the present invention may further include crystallizing the compound of formula (I) using at least one solvent, in particular a non-polar aprotic solvent selected from a cyclic alkane such as cyclohexane, an acyclic alkane such as heptane, an aromatic hydrocarbon such as toluene, and the like, and mixtures of the foregoing, preferably heptane.

Crystallization of the compound of formula (I) may be carried out according to a method conventionally used by a person skilled in the art. According to a particular embodiment, seeds of a compound of formula (I) are added to the product obtained from step (ii), optionally followed by a filtration step.

Said crystallization step may be followed by a further purification step, which in particular can consist in washing the crystallized product by an appropriate solvent, in particular one of the crystallization solvent as mentioned above, and for example heptane.

The final product of formula (I) may display a purity ranging from 95% to 100%, in particular from 98% to 100%, more particularly between 99% and 100%. Purity of the product may be determined by appropriate analytical techniques known to those of skill in the art, individually or in combination. Appropriate analytical techniques include high-performance liquid chromatography (HPLC, with detection by, for example, ultraviolet (UV) absorption, mass spectrometry, light scattering, and/or combinations thereof), gas chromatography (GC, with detection by, for example, flame-ionization detection, mass spectrometry, and/or combinations thereof), nuclear magnetic resonance (NMR, using any nuclide appropriate for the compound of formula (I), for example, ¹H, ¹³C, ¹⁹F, and/or combinations thereof), and trace element analysis techniques (such as inductively coupled plasma-mass spectrometry, flame-induced atomic absorption spectrometry, and the like).

According to a more particular aspect, the present invention relates to a method for preparing 8-chloro-N-(4-(trifluoromethoxy)phenyl)quinolin-2-amine also named (8-chloro-quinoline-2-yl)-(4-trifluoromethoxy-phenyl)-amine. Said compound is also known as ABX464:

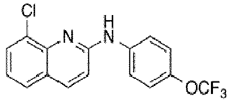

The method of preparation of ABX464 according to the present invention provides a product which is in a crystalline form which is named "Form I".

Said crystalline form "Form I" presents a melting point of 120.5°C (±2°C) and shows the following main peaks expressed as degree 2-Theta angles by a XRPD analysis: 7.3, 14.6, 23.5, and 28.4 (each time ±0.2) and may further show the following additional peaks: 12.1, 17.3, 18.4, 23.0; 24.2, 24.9, 27.4 and 29.1 (each time ±0.2) and even optionally further show the following additional peaks: 13.7, 16.3, 16.9, 18.1, 22.4, and 29.6 (each time ±0.2).

A characteristic X-ray powder diffractogram of Form I of 8-chloro-N-(4-(trifluoromethoxy)phenyl)quinolin-2-amine which was gently milled can be given in figure 1 and its characteristic signals are summarized in the following table :

**TABLE 1. Characteristic XRPD Signals of Crystalline Form I of 8-chloro-N-(4-(trifluoromethoxy)phenyl)quinolin-2-amine**

| **Angle (2-Theta) in degrees** | **Relative intensity** |
|---|---|
| (±0.2) | (%) |
| 7.3 | 100 |
| 12.1 | 9 |
| 13.7 | 7 |
| 14.6 | 68 |
| 16.3 | 11 |
| 16.9 | 10 |
| 17.3 | 18 |
| 18.1 | 9 |
| 18.4 | 53 |
| 22.4 | 37 |
| 23.0 | 41 |
| 23.5 | 57 |
| 24.2 | 34 |
| 24.9 | 40 |
| 27.4 | 26 |
| 28.4 | 76 |
| 29.1 | 29 |
| 29.6 | 14 |

According to a particular embodiment, a crystallisation step as defined above may be further implemented after the step (ii) in order to increase the purity of ABX464 under the "Form I".

In this particular case (that is to say when the compound of formula (I) is 8-chloro-N-(4-(trifluoromethoxy)phenyl)quinolin-2-amine), the compound of formula (III) implemented in the corresponding method of preparation according to the present invention is 4-trifluoromethoxyaniline. Still in this case, the compound of formula (II) implemented in the corresponding method of preparation according to the present invention is the following compound (3):

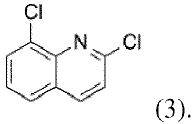

According to a particular aspect, the present invention thus relates to a preparation method of 8-chloro-N-(4-(trifluoromethoxy)phenyl)quinolin-2-amine comprising the steps of:
(1) reacting 4-trifluoromethoxyaniline with a compound of formula (3) as defined above to form the hydrochloride salt of the compound of formula (I) (that is to say the hydrochloride salt of 8-chloro-N-(4-(trifluoromethoxy)phenyl)quinolin-2-amine),
   wherein the molar ratio of the compound of formula (3) vs 4-trifluoromethoxyaniline is from 1.00:0.80 to 1.00:1.20, preferably from 1.00:0.80 to 1.00:1.10, and is for example 1.00:0.80, 1.00:0.85, 1.00:0.90, 1.00: 0.95, 1.00:1.00, 1.00:1.05, or 1.00:1.10, and no metal catalyst is present, in an organic solvent selected from the group consisting of ethyl acetate, isopropyl acetate, toluene, N,N-dimethylformamide (DMF), N-methyl-2-pyrrolidone (NMP), a (C₁-C₄)alcohol such as methanol, ethanol, isopropanol and butanol and the like, and mixtures of the foregoing, then
(2) recovering the compound of formula (I) in the form of a free base through addition of a base, preferably an inorganic base, and then
(3) optionally crystallizing the compound of formula (I) using at least one solvent selected from a cyclic alkane such as cyclohexane, an acyclic alkane such as heptane, an aromatic hydrocarbon such as toluene, and the like, and mixtures of the foregoing, and more particularly in heptane.

The addition of hydrochloric acid during step (i), of seeds of the hydrochloride salt of 8-chloro-N-(4-(trifluoromethoxy)phenyl)quinolin-2-amine during step (i) and/or of an acid near the beginning of step (i) as described above for the method of preparing a compound of formula (I), and more particularly of the three additions in combination, may similarly be implemented for the preparation of 8-chloro-N-(4-(trifluoromethoxy)phenyl)quinolin-2-amine, and more particularly during step (1) of the particular aspect as described above.

According to a more particular aspect, the present invention thus relates to a preparation method of 8-chloro-N-(4-(trifluoromethoxy)phenyl)quinolin-2-amine comprising the steps of:
(1) reacting 4-trifluoromethoxyaniline with a compound of formula (3) as defined above to form the hydrochloride salt of the compound of formula (I) (that is to say the hydrochloride salt of 8-chloro-N-(4-(trifluoromethoxy)phenyl)quinolin-2-amine),
   wherein the molar ratio of the compound of formula (3) vs 4-trifluoromethoxyaniline is from 1.00:0.80 to 1.00:1.20, preferably from 1.00:1.00 to 1.00:1.10, and is for example 1.00:0.80, 1.00:0.85, 1.00:0.90, 1.00: 0.95, 1.00:1.00, 1.00:1.05 or 1.00:1.10, and no metal catalyst is present, preferably in a solvent selected from (C₁-C₄)alcohol such as methanol, ethanol, isopropanol and butanol and the like, in particular in ethanol, isopropanol or butanol and more particularly in isopropanol,
(2) recovering the compound of formula (I) in the form of a free base through addition of a base, preferably an inorganic base, and
(3) optionally crystallizing the compound of formula (I) using at least one solvent selected from a cyclic alkane such as cyclohexane, an acyclic alkane such as heptane, an aromatic hydrocarbon such as toluene, and the like, and mixtures of the foregoing, and more particularly in heptane,
   wherein the hydrochloride salt of the compound of formula (I) (that is to say the hydrochloride salt of 8-chloro-N-(4-(trifluoromethoxy)phenyl)quinolin-2-amine) is isolated between step (1) and (2).

The addition of hydrochloric acid during step (i), of seeds of the hydrochloride salt of 8-chloro-N-(4-(trifluoromethoxy)phenyl)quinolin-2-amine during step (i) and/or of an acid near the beginning of step (i) as described above for the method of preparing a compound of formula (I), and more particularly of the three additions in combination, may similarly be implemented for the preparation of 8-chloro-N-(4-(trifluoromethoxy)phenyl)quinolin-2-amine, and more particularly during step (1) of the more particular aspect as described above.

According to a first variant, the present invention relates to a method of preparation of 8-chloro-N-(4-(trifluoromethoxy)phenyl)quinolin-2-amine (or ABX464), wherein the coupling step is carried out in a 1.00:0.80 molar ratio of the compound of formula (3) vs 4-trifluoromethoxyaniline, in a range of temperature between 70°C and 100°C, for 10 to 20 hours and in an organic solvent selected from ethyl acetate, isopropyl acetate, toluene, N,N-dimethylformamide (DMF), N-methyl-2-pyrrolidone (NMP), (C₁-C₄)alcohol such as methanol, ethanol, isopropanol and butanol and the like, and mixtures of the foregoing, in particular in ethanol, isopropanol or butanol, and more particularly in isopropanol.

According to a particular embodiment of this first variant, the present invention relates to a method of preparation of 8-chloro-N-(4-(trifluoromethoxy)phenyl)quinolin-2-amine (or ABX464), wherein the coupling step is carried out in a 1.00:0.80 molar ratio of the compound of formula (3) vs 4-trifluoromethoxyaniline, in a range of temperature between 80°C and 85°C, for 10 to 16 hours and in an organic solvent which is a (C₁-C₄)alcohol such as methanol, ethanol, isopropanol or butanol and the like, in particular in ethanol, isopropanol or butanol, and more particularly in isopropanol.

According to a second variant, the present invention relates to a method of preparation of 8-chloro-N-(4-(trifluoromethoxy)phenyl)quinolin-2-amine (or ABX464), wherein the coupling step is carried out in a 1.00:0.90 molar ratio of the compound of formula (3) vs 4-trifluoromethoxyaniline, in a range of temperature between 70°C and 100°C, for 10 to 20 hours and in an organic solvent selected from ethyl acetate, isopropyl acetate, toluene, N,N-dimethylformamide (DMF), N-methyl-2-pyrrolidone (NMP), (C₁-C₄)alcohol such as methanol, ethanol, isopropanol and butanol and the like, and mixtures of the foregoing, in particular in ethanol, isopropanol or butanol, and more particularly in isopropanol.

According to a particular embodiment of this second variant, the present invention relates to a method of preparation of 8-chloro-N-(4-(trifluoromethoxy)phenyl)quinolin-2-amine (or ABX464), wherein the coupling step is carried out in a 1.00:0.90 molar ratio of the compound of formula (3) vs 4-trifluoromethoxyaniline, in a range of temperature between 80°C and 85°C, for 10 to 16 hours and in an organic solvent which is a (C₁-C₄)alcohol such as methanol, ethanol, isopropanol or butanol and the like, in particular in ethanol, isopropanol or butanol, and more particularly in isopropanol.

According to a third variant, the present invention relates to a method of preparation of 8-chloro-N-(4-(trifluoromethoxy)phenyl)quinolin-2-amine (or ABX464), wherein the coupling step is carried out in a 1.00:0.95 molar ratio of the compound of formula (3) vs 4-trifluoromethoxyaniline, in a range of temperature between 70°C and 100°C, for 10 to 20 hours and in an organic solvent selected from ethyl acetate, isopropyl acetate, toluene, N,N-dimethylformamide (DMF), N-methyl-2-pyrrolidone (NMP), (C₁-C₄)alcohol such as methanol, ethanol, isopropanol and butanol and the like, and mixtures of the foregoing, in particular in ethanol, isopropanol or butanol, and more particularly in isopropanol.

According to a particular embodiment of this third variant, the present invention relates to a method of preparation of 8-chloro-N-(4-(trifluoromethoxy)phenyl)quinolin-2-amine (or ABX464), wherein the coupling step is carried out in a 1.00:0.95 molar ratio of the compound of formula (3) vs 4-trifluoromethoxyaniline, in a range of temperature between 80°C and 85°C, for 10 to 16 hours and in an organic solvent which is a (C₁-C₄)alcohol such as methanol, ethanol, isopropanol or butanol and the like, in particular in ethanol, isopropanol or butanol, and more particularly in isopropanol.

According to a fourth variant, the present invention relates to a method of preparation of 8-chloro-N-(4-(trifluoromethoxy)phenyl)quinolin-2-amine (or ABX464), wherein the coupling step is carried out in a 1.00:1.00 molar ratio of the compound of formula (3) vs 4-trifluoromethoxyaniline, in a range of temperature between 70°C and 100°C, for 10 to 20 hours and in an organic solvent selected from ethyl acetate, isopropyl acetate, toluene, N,N-dimethylformamide (DMF), N-methyl-2-pyrrolidone (NMP), (C₁-C₄)alcohol such as methanol, ethanol, isopropanol and butanol and the like, and mixtures of the foregoing, in particular in ethanol, isopropanol or butanol, and more particularly in isopropanol.

According to a particular embodiment of this fourth variant, the present invention relates to a method of preparation of 8-chloro-N-(4-(trifluoromethoxy)phenyl)quinolin-2-amine (or ABX464), wherein the coupling step is carried out in a 1.00:1.00 molar ratio of the compound of formula (3) vs 4-trifluoromethoxyaniline, in a range of temperature between 80°C and 85°C, for 10 to 16 hours and in an organic solvent which is a (C₁-C₄)alcohol such as methanol, ethanol, isopropanol or butanol and the like, in particular in ethanol, isopropanol or butanol, and more particularly in isopropanol.

According to a fifth variant, the present invention relates to a method of preparation of 8-chloro-N-(4-(trifluoromethoxy)phenyl)quinolin-2-amine (or ABX464), wherein the coupling step is carried out in a 1.00:1.10 molar ratio of the compound of formula (3) vs 4-trifluoromethoxyaniline, in a range of temperature between 70°C and 100°C, for 10 to 20 hours and in an organic solvent selected from ethyl acetate, isopropyl acetate, toluene, N,N-dimethylformamide (DMF), N-methyl-2-pyrrolidone (NMP), (C₁-C₄)alcohol such as methanol, ethanol, isopropanol and butanol and the like, and mixtures of the foregoing, in particular in ethanol, isopropanol or butanol, and more particularly in isopropanol.

According to a particular embodiment of this fifth variant, the present invention relates to a method of preparation of 8-chloro-N-(4-(trifluoromethoxy)phenyl)quinolin-2-amine (or ABX464), wherein the coupling step is carried out in a 1.00:1.10 molar ratio of the compound of formula (3) vs 4-trifluoromethoxyaniline, in a range of temperature between 80°C and 85°C, for 10 to 16 hours and in an organic solvent which is a (C₁-C₄)alcohol such as methanol, ethanol, isopropanol or butanol and the like, in particular in ethanol, isopropanol or butanol, and more particularly in isopropanol.

The addition of hydrochloric acid during step (i), of seeds of the hydrochloride salt of the compound of formula (I) during step (i) and/or of an acid near the beginning of step (i) as described above for the method of preparing a compound of formula (I), and more particularly of the three additions in combination, may be performed in each of the above-described particular variants and embodiments for preparing 8-chloro-N-(4-(trifluoromethoxy)phenyl)quinolin-2-amine.

According to a particular embodiment, the compound of formula (I) is 8-chloro-N-(4-(trifluoromethoxy)phenyl)quinolin-2-amine, and
- the compound of formula (III) as defined above is 4-trifluoromethoxyaniline,
- the compound of formula (II) as defined above is the following compound (3): 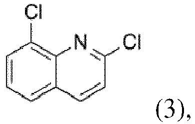
- the compound of formula (IV) as defined above is the following compound (2): 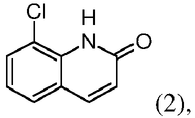
- the compound of formula (V) as defined above is the following compound (1): 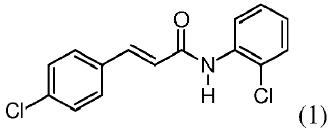
- the compound of formula (VI) as defined above is the following compound 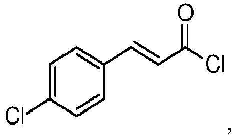
- the compound of formula (VI') as defined above is 2-chloro-aniline, and
- the compound of formula (VII) as defined above is the following compound

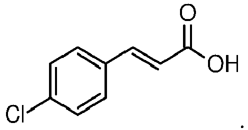

According to a particular aspect, the present invention further relates to a method of manufacturing a compound of formula (I) further comprising the step of preparing a pharmaceutical composition comprising such compound of formula (I), with pharmaceutically acceptable excipients.

Throughout the description, including the claims, the term "comprising a" should be understood as being synonymous with "comprising at least one", unless otherwise specified.

The expressions "between ... and ...", and "ranging from ... to ..." should be understood as meaning limits included, unless otherwise specified.

Hereinafter, the present invention will be described in more detail with reference to the following examples. These examples are provided to illustrate the present invention and should not be construed as limiting the scope and spirit of the present invention.

### EXAMPLES

### MATERIAL AND METHODS

### I. High-performance liquid chromatography (HPLC)

The apparatus HPLC is Agilent 1100.
HPLC Conditions:
HPLC column: Waters X-Bridge C18 3.5 µm, 100 x 2.1 mm
Temperature of the column : 40°C
Mobile phase:
A : H₂O/TFA (trifluoroacetic acid) 0.1%
B : ACN (acetonitrile)/TFA 0.1%
Gradient of elution :

| Time (minutes) | outflow (mL/min) | A (%) | B (%) | Curve |
|---|---|---|---|---|
| 1 | 0.3 | 95 | 5 | 6 |
| 17 | 0.3 | 2 | 98 | 6 |
| 19 | 0.3 | 2 | 98 | 6 |
| 20 | 0.3 | 95 | 5 | 6 |
| 27 | 0.3 | 95 | 5 | 6 |

Analysis time : 27 minutes
Injection volume: 5 µL
DAD or UV Detection: 220 nm
Sample Preparation: dilution water/ACN 50/50 at 0.3mg/mL

Determination of purity employs a reverse-phase HPLC with gradient elution, in which the intermediates are separated from other compounds in the sample and detected using a UV detector.

### II. ¹H-NMR,

The RMN apparatus is Bruker Avance 300.
¹H-NMR spectra are acquired using the solutions in CDCl₃ or DMSO-d6 according to European Pharmacopoeia method 2.2.33. The compound identity is confirmed based on chemical shifts and/or resonance signal intensity, and by comparison with the corresponding reference spectra.

### III. Differential Scanning Calorimetry (DSC)

- Netzsch DSC214 Polyma
- Al sealed sample pan (perforated lid)
- Atmosphere : Nitrogen
- Heating rate : 5°K/min (see below)
- Data treatment: Netzsch-TA Proteus ^{®} Software v 7.1.0.

The samples were analyzed by DSC from room temperature up to 135°C.

### IV. X-Rav Powder Diffraction (XRPD)

- Diffractometers Bruker D8 discover;
- Cupper anti cathode, tension 40 KV, intensity 40 mA
- θ/θ configuration, fixed sample
- Range of analysis : 3° to 30°
- Step increment: 0.04°
- Measuring time by step: 0.5s
- No internal reference
- Experimental treatment of the data by the EVA software (v 12.0)
The X-Ray peak positions and intensities are extracted from the analyzed samples.

### Example 1: Preparation of 8-chloro-N-(4-(trifluoromethoxy)phenyl)quinolin-2-amine (Form I), with a 1.00:0.90, 1.00:1.00, or 1.00:1.10 molar ratio of the compound of formula (3) vs 4-trifluoromethoxyaniline

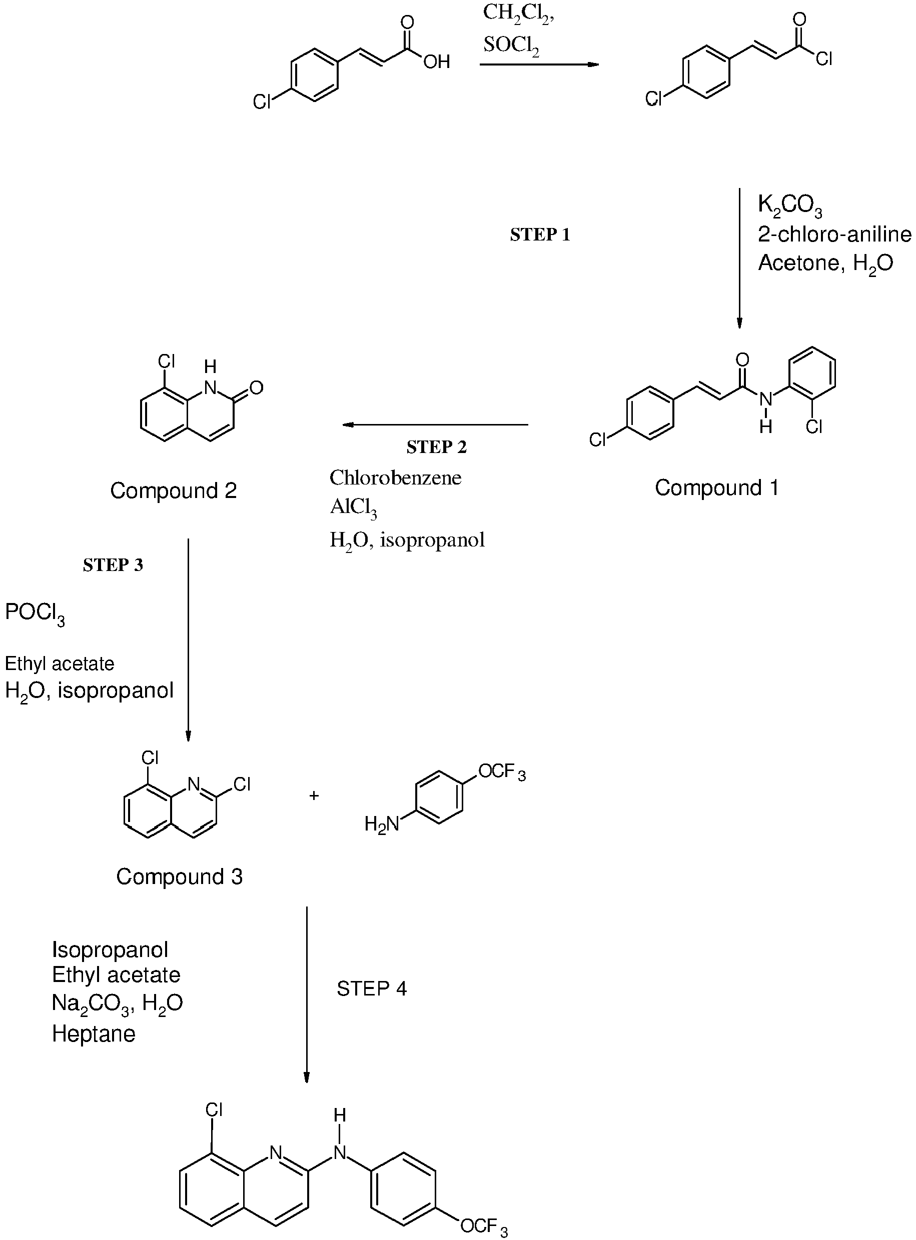

### Step 1

4-Chlorocinnamic acid (7.0 kg) and dichloromethane (28 L) were introduced into a reaction vessel under an inert atmosphere at ambient temperature. The mixture was heated to 30-40°C and thionyl chloride (5.0kg) was added. Temperature was raised to 40-50°C and stirring was pursued until complete dissolution. After reaction completion (HPLC) the reaction mixture was concentrated and cooled down to 15-25°C so as to obtain 4-chlorocinnamoyl chloride. The 4-chlorocinnamoyl chloride was added to a cooled mixture of potassium carbonate (8.0 kg) and 2-chloroaniline (5.1 kg) in acetone (14 L) and water (14 L). The reaction mixture was stirred for at least 6 more hours then cooled down again to 0-5°C and filtered. The solid was washed with water and dried under vacuum with a nitrogen flow to yield pure compound 1 (11.2 kg, 100% yield, HPLC 98.0%).

### Step 2

Under an inert atmosphere at ambient temperature, compound 1 (11.1 kg) was suspended into chlorobenzene (55 L). Aluminum chloride (15.0 kg) was added portionwise whilst stirring, and the suspension is then heated to 115-125°C. The reaction mixture is stirred for at least 2 more hours. After reaction completion (HPLC), the solution was cooled down to 30-40°C and poured onto a cooled mixture of water (89 L) and isopropanol (28 L) and stirred for 2 hours. The solid was filtered and washed with water (22 L) and isopropanol (22 L) and dried under vacuum with a nitrogen flow to yield pure compound 2 (4.9 kg, 73% yield, HPLC 99.9%).

### Step 3

Under an inert atmosphere at ambient temperature, compound 2 (4.6 kg) was suspended in phosphoryl chloride (9.9 kg). The suspension was heated to 115-125°C and stirred for at least 2 hours. Upon reaction completion (HPLC), the solution was cooled down to 40-50°C prior to dilution with ethyl acetate, and poured onto precooled (0-10°C) water (46 L). After at least 1 additional hour of stirring at that temperature, the compound 3 suspension was filtered. The solid was washed with water and isopropanol and dried under vacuum with a nitrogen flow, for at least 24 hours to yield pure compound 3 (4.6 kg, 89% yield, HPLC 99.9%).

### Step 4

Under an inert atmosphere at ambient temperature, compound 3 (4.6 kg, 1.0 eq.) was suspended in isopropanol (46 L). 4-(trifluoromethoxy)aniline (4.1 kg, 1.0 eq.) was added. The reaction mixture was then refluxed at 82°C for at least 12 hours. Upon reaction completion (¹H-NMR), the solution was cooled down to 0-10°C. The stirring was continued for at least a further 30 minutes prior to filtration. The resulting hydrochloride salt (solid) was washed with isopropyl alcohol and dried under vacuum with a nitrogen flow, at ambient temperature for at least 12 hours. The dried product was suspended in ethyl acetate (23 L), and stirred for at least 10 minutes at ambient temperature prior to the slow addition of a solution of sodium carbonate (2.9 kg) in water (23 L). After stirring for at least 30 minutes, the aqueous layer was removed and the organic layer was washed twice with water. Ethyl acetate was replaced by heptane (35 L) and the resulting solid was crystallized in heptane to yield pure crystalline Form I of ABX464 (6.4 kg, 82% yield, HPLC 100.0%).

This crystalline Form I is characterized by a powder X-ray diffractogram displaying peaks expressed as degree 2-Theta angles at 7.3, 14.6, 23.5 and 28.4 (each time ±0.2) of two-theta, and more particularly characterized by a powder X-ray diffractogram as illustrated in figure 1, and/or characterized by a single endotherm with an onset temperature of 120.5°C (±2°C) (Heating rate : 5°K/min in DSC method).

In an analogous manner, on approximately 30-gram scale, pure crystalline Form I of 8-chloro-N-(4-(trifluoromethoxy)phenyl)quinolin-2-amine was prepared with a yield of 70% using a 1.00:0.90 molar ratio of the compound of formula (3) vs 4-trifluoromethoxyaniline (step 4).

In an analogous manner, on approximately 30-gram scale, pure crystalline Form I of 8-chloro-N-(4-(trifluoromethoxy)phenyl)quinolin-2-amine was prepared with a yield of 84% using a 1.00:1.10 molar ratio of the compound of formula (3) vs 4-trifluoromethoxyaniline (step 4).

In an analogous manner, on approximately 30-gram scale, pure crystalline Form I of 8-chloro-N-(4-(trifluoromethoxy)phenyl)quinolin-2-amine was prepared with a yield of 51% using a 1.00:0.80 molar ratio of the compound of formula (3) vs 4-trifluoromethoxyaniline (step 4).

### Example 2: Preparation of hydrochloride salt of 8-chloro-N-(4-(trifluoromethoxy)phenyl)quinolin-2-amine in a step (i), with a 1.00:0.90 molar ratio of the compound of formula (3) vs 4-trifluoromethoxyaniline and trifluoroacetic acid

Under an inert atmosphere at ambient temperature, compound 3 (1 g, **1.0 eq.**) was suspended in isopropanol (10 mL) and trifluoroacetic acid (0.19 mL, 0.5 eq.). 4-(trifluoromethoxy)aniline (0.805 g, **0.9 eq.**) was added. The reaction mixture was then refluxed at 82°C for at least 5 hours leading to the formation at a level superior to 85% of conversion rate of hydrochloride salt of 8-Chloro-N-(4-(trifluoromethoxy)phenyl)quinolin-2-amine which can be isolated on solid form as described in example 1.

### Example 3: Preparation of hydrochloride salt of 8-Chloro-N-(4-(trifluoromethoxy)phenyl)quinolin-2-amine in a step (i), with a 1.00:1.00 molar ratio of the compound of formula (3) vs 4-trifluoromethoxyaniline, seeding with hydrochloride salt of 8-chloro-N-(4-(trifluoromethoxy)phenyl)quinolin-2-amine and addition of hydrochloric acid.

Under an inert atmosphere at ambient temperature, compound 3 (130 kg, **1.0 eq**.) was suspended in isopropanol (1 300 L). 4-(trifluoromethoxy)aniline (117 kg, **1.0 eq.**) was added. The reaction mixture was then refluxed at 82°C for at least 32 hours. After seeding with hydrochloride salt of 8-chloro-N-(4-(trifluoromethoxy)phenyl)quinolin-2-amine and upon reaction completion, the solution was cooled down to 0-10°C and hydrochloric acid (32.5 kg, **0.5 eq.**) was added. The stirring was continued for at least 1 hour prior to filtration. The resulting hydrochloride salt (solid) was washed with isopropyl alcohol and dried under vacuum with a nitrogen flow, at ambient temperature for at least 12 hours to yield pure hydrochloride salt of 8-chloro-N-(4-(trifluoromethoxy)phenyl)quinolin-2-amine (230 kg, 93% yield, HPLC 99,9%).

## Claims

1. A method for preparing a compound of formula (I) 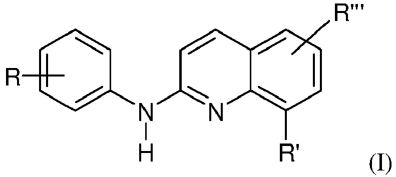 wherein
R is selected from a (C₁-C₃)alkyl group, in particular a methyl group, a (C₁-C₃)alkoxy group, in particular a methoxy group, a (C₁-C₃)fluoroalkyl group, in particular a trifluoromethyl group, a halogen atom and more particularly a fluorine or chlorine atom, a (C₁-C₃)fluoroalkoxy group, in particular a trifluoromethoxy group and a -NR₁R₂ group, in particular an amino group,
R' represents a halogen atom and more particularly a fluorine or chlorine atom or a methyl group, and
R"' represents a hydrogen atom or a 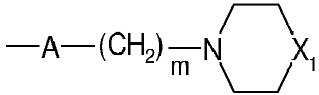 group, wherein A is O or NH, m is 2 or 3 and X₁ is -O-, -CH₂- or -N(Ra)-, where Ra is a (C₁-C₃)alkyl group, in particular a methyl group, and
R₁ and R₂ are independently a hydrogen atom or a (C₁-C₃)alkyl group,
the method comprising the following steps:
- (i) reacting a compound of formula (II) 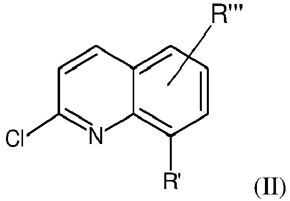 wherein R' and R'" are as defined above,
with a compound of formula (III) 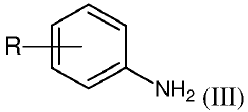 wherein
R is as defined above,
to form the hydrochloride salt of the compound of formula (I),
wherein the molar ratio of the compound of formula (II) vs. the compound of formula (III) is from 1.00:0.80 to 1.00:1.20, and no metal catalyst is present and then
- (ii) recovering the compound of formula (I) in the form of a free base through addition of a base.

2. The method according to claim 1, wherein the compound of formula (II) is prepared by chlorination of a compound of formula (IV) 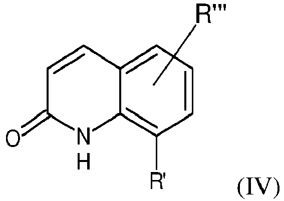 wherein
R' represents a halogen atom and more particularly a fluorine or chlorine atom or a methyl group, and
R"' represents a hydrogen atom or a 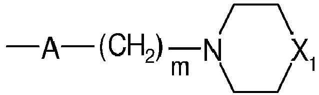 group, wherein A is O or NH, m is 2 or 3 and X₁ is -O-, -CH₂- or -N(Ra)-, where Ra is a (C₁-C₃)alkyl group, in particular a methyl group.

3. The method according to claim 2, wherein the compound of formula (IV) is prepared by cyclizing a compound of formula (V) 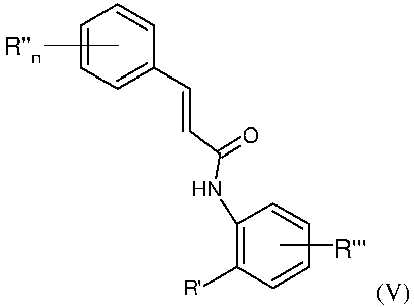 wherein
R' represents a halogen atom and more particularly a fluorine or chlorine atom or a methyl group, and
R"' represents a hydrogen atom or a 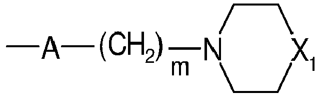 group, wherein A is O or NH, m is 2 or 3 and X₁ is -O-, -CH₂- or -N(Ra)-, where Ra is a (C₁-C₃)alkyl group, in particular a methyl group,
R" represents a halogen atom, in particular a chlorine atom, a (C₁-C₃)alkyl group, in particular a methyl group, and
n is 1 or 2.

4. The method according to claim 3, wherein the compound of formula (V) is prepared by amidation of a compound of formula (VI) 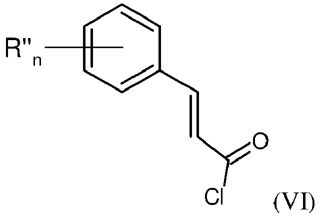 wherein
R" represents a halogen atom, in particular a chlorine atom, a (C₁-C₃)alkyl group, in particular a methyl group, and
n is 1 or 2,
with a compound of formula (VI') 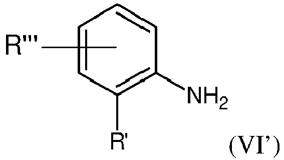 wherein
R' represents a halogen atom and more particularly a fluorine or chlorine atom or a methyl group, and
R"' represents a hydrogen atom or a 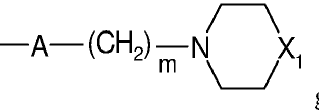 group, wherein A is O or NH, m is 2 or 3 and X₁ is -O-, -CH2- or -N(Ra)-, where Ra is a (C₁-C₃)alkyl group, in particular a methyl group.

5. The method according to claim 4, wherein the compound of formula (VI) is prepared by conversion of a carboxylic acid function of a compound of formula (VII) 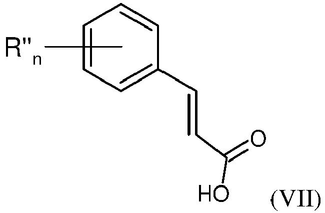 wherein
R" represents a halogen atom, in particular a chlorine atom, a (C₁-C₃)alkyl group, in particular a methyl group, and
n is 1 or 2,
into an acyl chloride function.

6. The method for preparing a compound of formula (I) according to claim 1, wherein it comprises at least the following steps:
- Step (A) : conversion of a carboxylic acid function of a compound of formula (VII) 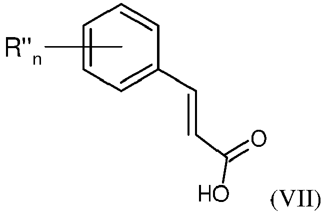 wherein R" represents a halogen atom, in particular a chlorine atom, a (C₁-C₃)alkyl group, in particular a methyl group, and n is 1 or 2,
into an acyl chloride function,
to prepare a compound of formula (VI) 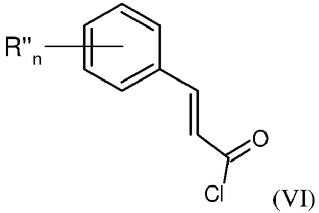 wherein R" represents a halogen atom, in particular a chlorine atom, a (C₁-C₃)alkyl group, in particular a methyl group, and n is 1 or 2;
- Step (B): amidation of a compound of formula (VI) 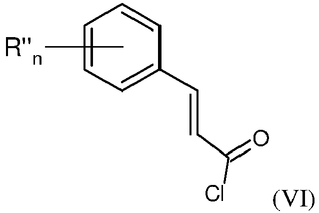 wherein R" represents a halogen atom, in particular a chlorine atom, a (C₁-C₃)alkyl group, in particular a methyl group, and n is 1 or 2,
with a compound of formula (VI') 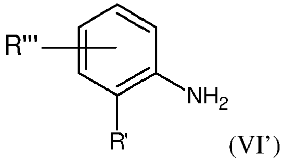
wherein
R' represents a halogen atom and more particularly a fluorine or chlorine atom or a methyl group, and
R"' represents a hydrogen atom or a 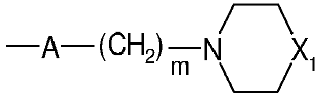 group, wherein A is O or NH, m is 2 or 3 and X₁ is -O-, -CH2- or -N(Ra)-, where Ra is a (C₁-C₃)alkyl group, in particular a methyl group,
to prepare a compound of formula (V) 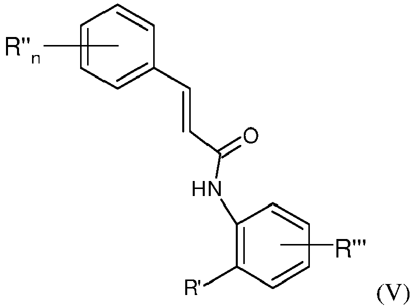 wherein
R' represents a halogen atom and more particularly a fluorine or chlorine atom or a methyl group, and
R"' represents a hydrogen atom or a 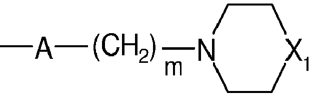 group, wherein A is O or NH, m is 2 or 3 and X₁ is -O-, -CH₂- or -N(Ra)-, where Ra is a (C₁-C₃)alkyl group, in particular a methyl group, and
R" represents a halogen atom, in particular a chlorine atom, a (C₁-C₃)alkyl group, in particular a methyl group, and
n is 1 or 2;
- Step (C): cyclizing a compound of formula (V) 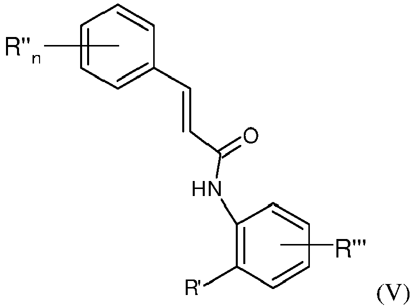 wherein
R' represents a halogen atom and more particularly a fluorine or chlorine atom or a methyl group, and
R"' represents a hydrogen atom or a 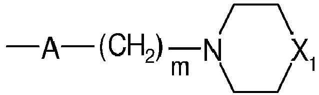 group, wherein A is O or NH, m is 2 or 3 and X₁ is -O-, -CH₂- or -N(Ra)-, where Ra is a (C₁-C₃)alkyl group, in particular a methyl group,
R" represents a halogen atom, in particular a chlorine atom, a (C₁-C₃)alkyl group, in particular a methyl group, and
n is 1 or 2,
to prepare a compound of formula (IV) 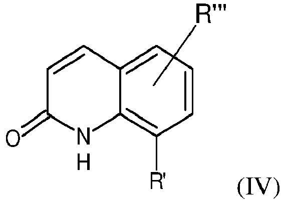 wherein
R' represents a halogen atom and more particularly a fluorine or chlorine atom or a methyl group, and
R"' represents a hydrogen atom or a 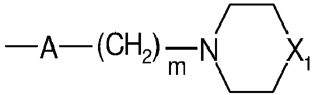 group, wherein A is O or NH, m is 2 or 3 and X₁ is -O-, -CH₂- or -N(Ra)-, where Ra is a (C₁-C₃)alkyl group, in particular a methyl group,
- Step (D): chlorination of a compound of formula (IV) 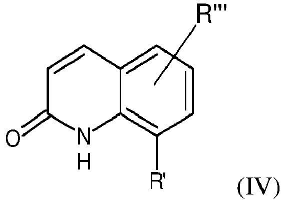 wherein
R' represents a halogen atom and more particularly a fluorine or chlorine atom or a methyl group, and
R"' represents a hydrogen atom or a 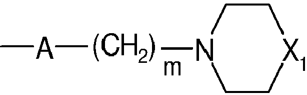 group, wherein A is O or NH, m is 2 or 3 and X₁ is -O-, -CH₂- or -N(Ra)-, where Ra is a (C₁-C₃)alkyl group, in particular a methyl group,
to prepare a compound of formula (II) 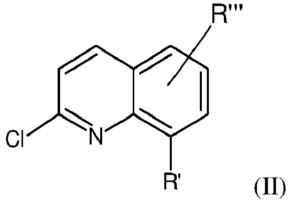 wherein
R' represents a halogen atom and more particularly a fluorine or chlorine atom or a methyl group, and
R"' represents a hydrogen atom or a 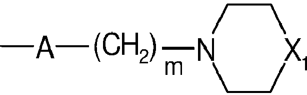 group, wherein A is O or NH, m is 2 or 3 and X₁ is -O-, -CH₂- or -N(Ra)-, where Ra is a (C₁-C₃)alkyl group, in particular a methyl group;
- Step (E):
∘ (i) reacting a compound of formula (II) 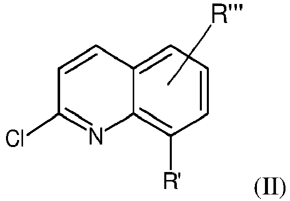 wherein
R' represents a halogen atom and more particularly a fluorine or chlorine atom or a methyl group, and
R"' represents a hydrogen atom or a 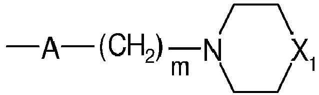 group, wherein A is O or NH, m is 2 or 3 and X₁ is -O-, -CH₂- or -N(Ra)-, where Ra is a (C₁-C₃)alkyl group, in particular a methyl group,
with a compound of formula (III) 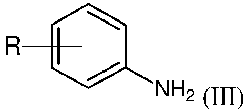 wherein
R is selected from a (C₁-C₃)alkyl group, in particular a methyl group, a (C₁-C₃)alkoxy group, in particular a methoxy group, a (C₁-C₃)fluoroalkyl group, in particular a trifluoromethyl group, a halogen atom and more particularly a fluorine or chlorine atom, a (C₁-C₃)fluoroalkoxy group, in particular a trifluoromethoxy group and a -NR₁R₂ group, in particular an amino group,
R₁ and R₂ are independently a hydrogen atom or a (C₁-C₃)alkyl group,
to form the hydrochloride salt of the compound of formula (I), wherein the molar ratio of the compound of formula (II) vs. the compound of formula (III) is from 1.00:0.80 to 1.00:1.20, and no metal catalyst is present and then
∘ (ii) recovering the compound of formula (I) in the form of a free base through addition of a base.

7. The method for preparing a compound of formula (I) according to any one of the preceding claims, wherein hydrochloric acid is added during step (i) as defined in claim 1 or step (E) as defined in claim 6.

8. The method for preparing a compound of formula (I) according to any one of the preceding claims, wherein seeds of the hydrochloride salt of the compound of formula (I) are added during step (i) as defined in claim 1 or part (i) of step (E) as defined in claim 6.

9. The method for preparing a compound of formula (I) according to any one of the preceding claims, wherein an acid is added near the beginning of step (i) as defined in claim 1 or part (i) of step (E) as defined in claim 6, said acid being for example selected from hydrochloric acid, hydrobromic acid, sulfuric acid, perchloric acid, phosphoric acid, trifluoroacetic acid, acetic acid, citric acid, oxalic acid, maleic acid, tartaric acid, succinic acid, malonic acid and mixtures thereof, in particular from phosphoric acid, hydrochloric acid, trifluoroacetic acid and mixtures thereof.

10. The method for preparing a compound of formula (I) according to any one of the preceding claims, wherein the molar ratio of the compound of formula (II) vs. the compound of formula (III) is from 1.00:0.80 to 1.00:1.10, and is for example 1.00:0.80, 1.00:0.85, 1.00:0.90, 1.00: 0.95, 1.00:1.00, 1.00:1.05, or 1.00:1.10.

11. The method for preparing a compound of formula (I) according to any one of the preceding claims, wherein step (i) as defined in claim 1 or part (i) of step (E) as defined in claim 6 is carried out in an organic solvent selected from the group consisting of ethyl acetate, isopropyl acetate, toluene, N,N-dimethylformamide (DMF), N-methyl-2-pyrrolidone (NMP), a (C₁-C₄)alcohol such as methanol, ethanol, isopropanol and butanol and the like, and mixtures of the foregoing, and is more particularly selected from the group consisting of ethanol, butanol and isopropanol, and is even more preferably isopropanol and/or
wherein step (i) as defined in claim 1 or part (i) of step (E) as defined in claim 6 is carried out at a reaction temperature ranging from 60°C to 120°C, preferably from 70°C to 100°C, and most preferably from 80°C to 85°C.

12. The method for preparing a compound of formula (I) according to any one of the preceding claims, wherein the base used in step (ii) as defined in claim 1 or step (ii) of step (E) as defined in claim 6 is selected from a group consisting of an organic base such as pyridine, triethylamine, diisopropylamine, and the like, and an inorganic base such as sodium carbonate (Na₂CO₃), sodium bicarbonate (NaHCO₃), potassium carbonate (K2CO3), potassium bicarbonate (KHCO₃), sodium hydroxide (NaOH), potassium hydroxide (KOH), lithium hydroxide (LiOH), and the like, in particular an inorganic base such as sodium carbonate (Na₂CO₃), sodium bicarbonate (NaHCO₃), potassium carbonate (K2CO3), sodium hydroxide (NaOH), potassium hydroxide (KOH), and the like.

13. The method for preparing a compound of formula (I) according to any one of the preceding claims, wherein the method further comprises purification steps, and for example isolating the hydrochloride salt of the compound of formula (I) and/or filtration steps between step (i) and step (ii) as defined in claim 1 or between step (i) and step (ii) of step (E) as defined in claim 6,
and/or
wherein the method further includes crystallizing the compound of formula (I) using at least one solvent, in particular a non-polar aprotic solvent selected from a cyclic alkane such as cyclohexane, an acyclic alkane such as heptane, an aromatic hydrocarbon such as toluene, and the like, and mixtures of the foregoing, preferably heptane.

14. The method for preparing a compound of formula (I) according to any one of claims 5 to 13, wherein the conversion of a carboxylic function into an acyl chloride function as defined in claim 5 or step (A) as defined in claim 6 is carried out by using a chlorination agent selected from a group consisting of SOCl₂, PCl₃, POCl₃, PCl₅, and the like, preferably SOCl₂, and optionally in a solvent such as dichloromethane.

15. The method for preparing a compound of formula (I) according to any one of claims 4 to 14, wherein the amidation as defined in claim 4 or step (B) as defined in claim 6 is carried out by using a base selected from a group consisting of an organic base such as pyridine, triethylamine, diisopropylamine and the like, and an inorganic base such as sodium carbonate (Na₂CO₃), sodium bicarbonate (NaHCO₃), potassium carbonate (K2CO3), potassium bicarbonate (KHCO₃), sodium hydroxide (NaOH), potassium hydroxide (KOH), lithium hydroxide (LiOH), and the like, in particular an inorganic base such as sodium carbonate (Na₂CO₃), sodium bicarbonate (NaHCO₃), potassium carbonate (K2CO3), sodium hydroxide (NaOH), potassium hydroxide (KOH), and the like, and preferably potassium carbonate.

16. The method for preparing a compound of formula (I) according to any one of claims 3 to 15, wherein the cyclizing as defined in claim 3 or step (C) as defined in claim 6 is carried out by using chlorobenzene, trifluorotoluene, fluorobenzene, toluene, dichloroethane or the like, and mixtures of the foregoing, and a Lewis acid selected from aluminum chloride (AlCl₃), BF₃, trifluoromethanesulfonic acid, titanium chloride, methanesulfonic acid and the like, preferably AlCl₃.

17. The method for preparing a compound of formula (I) according to any one of claims 2 to 16, wherein the chlorination as defined in claim 2 or step (D) as defined in claim 6 is carried out by using a chlorination agent selected from a group consisting of SOCl₂, PCl₃, POCl₃, PCl₅, and the like, preferably POCl₃, and optionally by using a solvent such as ethyl acetate, acetonitrile, toluene, dichloromethane.

18. The method for preparing a compound of formula (I) according to any one of the preceding claims, wherein the compound of formula (I) is 8-chloro-N-(4-(trifluoromethoxy)phenyl)quinolin-2-amine, the compound of formula (III) according to claim 1 is 4-trifluoromethoxyaniline and the compound of formula (II) according to claim 1 is the following compound (3):

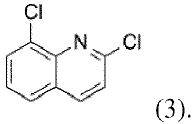

19. The method for preparing a compound of formula (I) according to any one of the preceding claims, wherein
- the compound of formula (I) is 8-chloro-N-(4-(trifluoromethoxy)phenyl)quinolin-2-amine,
- the compound of formula (III) as defined in claim 1 or claim 6 is 4-trifluoromethoxyaniline,
- the compound of formula (II) as defined in claim 1 or claim 6 is the following compound (3): 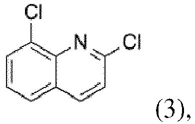
- the compound of formula (IV) as defined in claim 2 or claim 6 is the following compound (2): 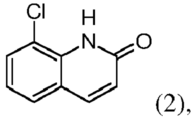
- the compound of formula (V) as defined in claim 3 or claim 6 is the following compound (1): 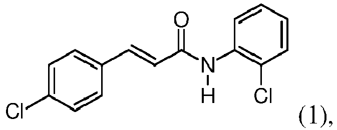
- the compound of formula (VI) as defined in claim 4 or claim 6 is the following compound 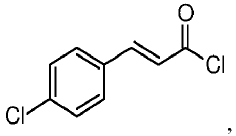
- the compound of formula (VI') as defined in claim 4 or claim 6 is 2-chloro-aniline, and
- the compound of formula (VII) as defined in claim 5 or claim 6 is the following compound

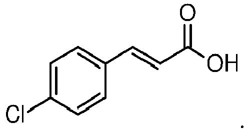

20. The method for preparing a compound of formula (I) according to any one of the preceding claims, wherein the compound of formula (I) is 8-chloro-N-(4-(trifluoromethoxy)phenyl)quinolin-2-amine in crystalline Form I, **characterized by** (1) a melting point of 120.5°C (±2°C), and/or (2) an XRPD analysis showing (a) the following main peaks expressed as degree 2-Theta angles by a XRPD analysis: 7.3, 14.6, 23.5, and 28.4 (each time ±0.2), optionally further showing the following additional peaks: 12.1, 17.3, 18.4, 23.0; 24.2, 24.9, 27.4 and 29.1 (each time ±0.2), and even optionally further showing the following additional peaks: 13.7, 16.3, 16.9, 18.1, 22.4, and 29.6 (each time ±0.2); (b) the signals listed in Table 1; or (c) a spectrum substantially the same as FIG. 1.

21. The method for preparing a compound of formula (I) according to any one of the preceding claims, further comprising the step of preparing a pharmaceutical composition comprising such compound of formula (I), with pharmaceutically acceptable excipients.
